# EUROPÄISCHE PATENTSCHRIFT

(11) **EP 1 350 838 B1**
(45) Veröffentlichungstag und Bekanntmachung des Hinweises auf die Patenterteilung: **22.02.2017**
(21) Anmeldenummer: 03007097.3
(22) Anmeldetag: 28.03.2003
(51) Int. Cl.: C12N 1/11, C12N 15/79, C12P 21/02, C07K 14/765

(54) **EXPRESSION VON REKOMBINANTEN HUMANEN PROTEINEN IN TETRAHYMENA**
EXPRESSION OF RECOMBINANT HUMAN PROTEINS IN TETRAHYMENA
EXPRESSION DE PROTÉINES HUMAINES RECOMBINÉES DANS TETRAHYMENA

(30) Priorität: 30.03.2002 DE 10214413
(43) Veröffentlichungstag der Anmeldung: 08.10.2003
(73) Patentinhaber: Lonza Ltd, 4002 Basel (CH)
(72) Erfinder: KIY, Thomas, 65929 Frankfurt/Main (DE); RÜSING, Matthias, 50935 Köln (DE)
(74) Vertreter: Michalski Hüttermann & Partner Patentanwälte mbB

(56) Entgegenhaltungen:
- WO-A-00/46381
- WO-A-98/01572
- GAERTIG J: "Suface display of a parasite antigen in the ciliate Tetrahymena thermophila" NATURE BIOTECHNOLOGY, NATURE PUBLISHING, US, Bd. 17, Nr. 5, 17. Mai 1999 (1999-05-17), Seiten 462-465, XP002141865 ISSN: 1087-0156
- DUGAICZYK A ET AL: "NUCLEOTIDE SEQUENCE AND THE ENCODED AMINO-ACIDS OF HUMAN SERUM ALBUMIN MESSENGER RNA" PROCEEDINGS OF THE NATIONAL ACADEMY OF SCIENCES OF THE UNITED STATES, Bd. 79, Nr. 1, 1982, Seiten 71-75, XP009014860 1982 ISSN: 0027-8424
- BRUNS P J ET AL: "Biolistic transformation of macro- and micronuclei." METHODS IN CELL BIOLOGY. UNITED STATES 2000, Bd. 62, 2000, Seiten 501-512, XP001154231 ISSN: 0091-679X
- TANIGUCHI T ET AL: "CARBOHYDRATES OF LYSOSOMAL ENZYMES SECRETED BY TETRAHYMENA-PYRIFORMIS" JOURNAL OF BIOLOGICAL CHEMISTRY, Bd. 260, Nr. 26, 1985, Seiten 13941-13946, XP009014847 ISSN: 0021-9258

<110> Celanese ventures GmbH
<120> Expression von rekombinanten Proteinen in Tetrahymena
<130> ax01006de1
<160> 8
<170> PatentIn version 3.1
<210> 1
   <211> 30
   <212> DNA
   <213> Künstliche sequenz
<400> 1
   ggcacaatgc attgggtaac ctttattagc 30
<210> 2
   <211> 34
   <212> DNA
   <213> Künstliche Sequenz
<400> 2
   aaatgggatc ctcataagcc taaggcagct tgac 34
<210> 3
   <211> 29
   <212> DNA
   <213> Künstliche Sequenz
<400> 3
   aaaaataaaa aagtttgaaa aaaaaccttc 30
<210> 4
   <211> 22
   <212> DNA
   <213> Künstliche Sequenz
<400> 4
   gtttagctga ccgattcagt tc 22
<210> 5
   <211> 54
   <212> DNA
   <213> Homo sapiens
<400> 5
   atgaagtggg taacctttat ttcccttctt tttctcttta gctcggctta ttcc 54
<210> 6
   <211> 18
   <212> PRT
   <213> Homo sapiens
<400> 6
   Met Lys Trp Val Thr Phe Ile Ser Leu Leu Phe Leu Phe Ser Ser Ala
   1               5                   10                  15
   Tyr Ser
<210> 7
   <211> 18
   <212> DNA
   <213> Homo sapiens
<400> 7
   aggggtgtgt ttcgtcga 18
<210> 8
   <211> 6
   <212> PRT
   <213> Homo sapiens
<400> 8
   Arg Gly Val Phe Arg Arg
   1               5

## Beschreibung

Die vorliegende Erfindung betrifft die rekombinante Expression von humanen Proteinen in *Tetrahymena.*

Die Produktion von rekombinanten Proteinen durch heterologe Proteinexpression stellt eine Alternative zur Gewinnung von Proteinen aus natürlichen Quellen dar. Natürliche Quellen für Proteine z. B. als Pharmazeutika sind oft limitiert, sehr teuer in der Reinigung oder stehen einfach nicht zur Verfügung. Außerdem können sie durch die Gefahr von toxischen oder insbesondere infektiösen Verunreinigungen sehr problematisch sein. Die Bio- und Gentechnologie ermöglicht dagegen heute eine wirtschaftliche und sichere Produktion von einer ganzen Reihe von Proteinen in ausreichenden Mengen mittels heterologer Expression für verschiedenste Einsatzgebiete: z. B. Antikörper (für die Diagnose, passive Immunisierung und Forschung), Hormone (wie Insulin, Erythropoietin (EPO), Interleukine usw. für den therapeutischen Einsatz), Enzyme (z. B. für den Einsatz in der Lebensmitteltechnologie, Diagnose, Forschung), Blutfaktoren (zur Behandlung von Hämophilie), Impfstoffe usw. (Glick & Pasternak 1998, Molecular Biotechnology, ASM Press, Washington DC, Chapter 10: 227-252).

Humane Proteine für einen medizinischen Einsatz müssen in ihren biochemischen, biophysikalischen und funktionalen Eigenschaften identisch mit dem natürlichen Protein sein. Bei einer rekombinanten Herstellung solcher Proteine durch heterologe Genexpression ist dabei zu beachten, dass es im Gegensatz zu Bakterien in eukaryotischen Zellen eine Reihe von posttranslationalen Proteinmodifizierungen gibt, beispielsweise die Ausbildung von Disulfidbrücken, proteolytische Spaltung von Vorläuferproteinen, Modifikationen von Aminosäureresten, wie Phosphorylierung, Acetylierung, Acylierung, Sulfatisierung, Carboxylierung, Myristylierung, Palmitylierung und insbesondere Glycosylierungen. Darüber hinaus werden Proteine in eukaryotischen Zellen oft erst durch einen komplexen Mechanismus unter Beteiligung von Chaperonen in die richtige dreidimensionale Struktur gebracht. Diese Modifizierungen spielen eine sehr wichtige Rolle hinsichtlich der spezifischen strukturellen und funktionalen Eigenschaften der Proteine, wie etwa der Aktivität von Enzymen, der Spezifität (Rezeptorbindung, Zellerkennung), Faltung, Löslichkeit etc. des Proteins (Ashford & Platt 1998, in: Post-Translational Processing - A Practical Approach, Ed. Higgins & Hames, Oxford University Press, Chapter 4: 135-174; Glick & Pasternak 1998, Molecular Biotechnology, ASM Press, Washington DC, Chapter 7: 145-169).

Von der natürlichen Struktur abweichende Modifizierungen können z. B. zur Inaktivierung der Proteine führen oder ein hohes allergenes Potential besitzen.

So führt etwa die Produktion von humanem Serum Albumin (HSA) in Bakterien häufig zu nicht korrekt gefalteten unlöslichen Proteinen, da benötigte Chaperone fehlen und Disulfidbrücken nicht korrekt gebildet werden. Als Alternativen zur Gewinnung von natürlichem HSA aus menschlichem Spenderblut sind daher verschiedene eukaryotische Produktionssysteme für die Gewinnung von rekombinantem HSA in der Entwicklung. Das Spektrum reicht dabei von der Expression in Pflanzen über Pilze bis hin zu transgenen Tieren oder Säugerzellkulturen.

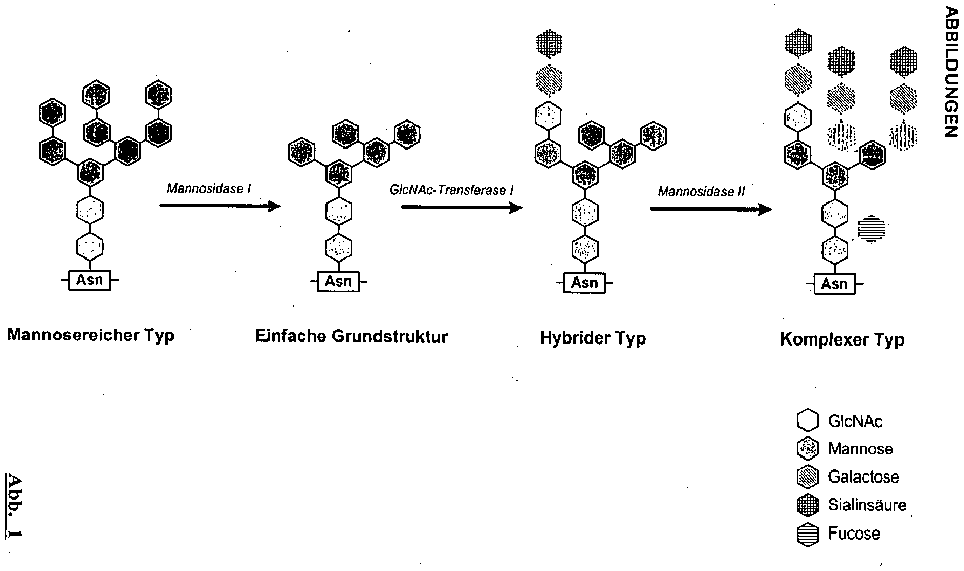

Obwohl eine ganze Reihe von bakteriellen und eukaryotischen Expressionssystemen für die Produktion von rekombinanten Proteinen etabliert sind, gibt es kein universelles System, welches das ganze Spektrum der - insbesondere bei eukaryotischen Proteinen - möglichen Proteinmodifikationen abdeckt und damit universell einsetzbar wäre (Castillo 1995, Bioprocess Technology 21: 13-45; Geise et al. 1996, Prot. Expr. Purif. 8: 271-282; Verma et al. 1998 J. Immunological Methods 216: 165-181; Glick & Pasternak 1998, Molecular Biotechnology, ASM Press, Washington DC, Chapter 7: 145-169). Weiterhin äusserst problematisch ist aber auch, dass einige der vielfach genutzten Systeme zu ungewöhnlichen und teilweise unerwünschten posttranslationalen Proteinmodifikationen führen. So sind rekombinant exprimierte Proteine aus Hefen mitunter extrem stark mit Mannoseresten glycosyliert (siehe Abb. 1: Proteinglycosylierung). Diese sogenannten "high Mannose" Strukturen aus Hefen bestehen dabei aus ca. 8-50 Mannoseresten und unterscheiden sich damit erheblich von den mannosereichen Glycoproteinstrukturen aus Säugetierzellen, die maximal 5-9 Mannosereste aufweisen (Moremen et al. 1994, Glycobiology 4(2): 113-125). Diese hefetypischen Mannosestrukturen sind starke Allergene und somit bei der Produktion von rekombinanten Glycoproteinen für einen therapeutischen Einsatz sehr problematisch (Tuite et al. 1999, in Protein Expression - A Practical Approach, Ed. Higgins & Hames, Oxford University Press, Chapter 3: insbesondere Seite 76). Ausserdem können in Hefen keine hybriden oder komplexen Glycoproteinstrukturen gebildet werden, was den Einsatz als Expressionssystem weiter einschränkt. Pflanzen, die in letzter Zeit immer öfter als Produktionssysteme für rekombinante Proteine diskutiert und genutzt werden, weisen dagegen statt der für Säuger typischen Sialinsäure auf den Glykoproteinstrukturen Xylose auf (Ashford & Platt, s.o.). Xylose und die ebenfalls bei Pflanzen nachgewiesene alpha -1,3 verknüpfte Fucose können ein allergisches Risiko darstellen und sind somit auch problematisch (Jenkins et al. 1996, Nature Biotech. 14: 975-981).

Deshalb besteht gerade für neue eukaryotische Expressionssysteme ein grosser Bedarf, vor allem als Alternative zu der sehr kostenintensiven und aufwendigen Herstellung von rekombinanten Proteinen durch Säugerkulturzellen.

Ein solches System sollte idealerweise folgende Anforderungen erfüllen:
1) Es müssen Selektionsmarker und regulative DNA-Elemente (wie Transkriptions- und Translationssignale etc.) zur Verfügung stehen.
2) Das Expressionssystem sollte wichtige eukaryotische posttranslationale Proteinmodifizierungen aufweisen, dabei aber keine Allergene für den Menschen hervorbringen.
3) Die Herstellung der rekombinanten Proteine sollte möglichst einfach und wirtschaftlich sein, z. B. durch Fermentation der Zellen oder Organismen im Produktionsmassstab (der mehrere 1000 L betragen kann) auf einfachen Medien und einfache Aufarbeitung der Produkte. Für letzteres äusserst vorteilhaft wäre die Möglichkeit zur Sekretion der exprimierten Proteine durch die Zellen. So ist eine Abtrennung der Zellen vom produkthaltigen Kulturüberstand leicht möglich und das Ausmass der Verunreinungen, die bei der Aufarbeitung vom Produkt zu trennen sind, reduziert sich massgeblich, wenn auf einen Zellaufschluss gänzlich verzichtet werden kann.

Zu den bereits etablierten eukaryotischen Expressionssystemen wie z. B. Hefe-Säuger- oder Insektenkulturzellen könnten Protozoen oder Protisten (als Definition siehe z.B. Hendersons's dictionary of biological terms, 10th ed. 1989, Eleanor Lawrence, Longman Scientific & Technical, England, oder Margulis et al. (Eds) 1990. Handbook of Protoctista, Jones & Bartlett, Boston; van den Hoek et al. 1995, Algae - An introduction to phycology, Cambridge University Press) als Expressionssysteme eine interessante Alternative bieten. Bei diesen Organismen handelt es sich um eine sehr heterogene Gruppe von eukaryotischen, in der Regel einzellagen. Mikroorganismen. Sie besitzen die für eukaryotische Zellen typische Kompartimentierung und Differenzierung. Einige sind relativ nah verwand mit höheren Eukaryoten, andererseits ähneln sie aber teilweise in Kultivierung und Wachstum eher Hefen oder sogar Bakterien und lassen sich relativ leicht bei hoher Zelldichte auf einfachen Medien im grossen Massstab fermentieren.

Für einige wenige Protisten oder Protozoen sind Methoden zur Transformation und heterologen Proteinexpression beschrieben. Insbesondere in parasitischen Protozoen, wie *Trypanosoma*, *Leishmania, Plasmodium* u.a. wurden verschiedene Versuche zur Transformation und Expression rekombinanter Proteine unternommen (Beverly 2000, WO 00/58483 A2). Eine Übersicht darüber gibt Kelly (1997, Adv. in Parasitol., Vol 39, 227-270). Die Möglichkeit zur heterologen Proteinexpression wurde auch in einer Reihe weiterer Protisten (eukaryotischen Mikroorganismen) gezeigt, wie z. B. im Schleimpilz *Dictyostelium discoideum* (Manstein et al. 1995, Gene 162: 129-134, Jung und Williams 1997, Biotechnol. Appl. Biochem. 25: 3-8), in Ciliaten (WO 98/01572 A1) wie *Paramecium* (Boileau et al. 1999, J. Eukaryot. Microbiol. 46: 56-65) und *Tetrahymena* (Gaertig et al. 1999, Nature Biotech. 17: 462-465, WO 00/46381 A1). Auch in photoautotrophen Protisten, den Mikroalgen, konnten Proteine heterolog exprimiert werden. Hier seien z.B. *Chlamydomonas* (Hall et al. 1993, Gene 124: 75-81), *Volvox* (Schiedlmeier et al. 1994, PNAS 91: 5080-5084), Dinoflagellaten (ten Lohuis & Miller 1998, Plant Journal 13: 427-435) und Diatomeen (Dunahay et al. 1995, J. Phycol. 31: 1004-1012) genannt. In den meisten Fällen wurden dabei jedoch lediglich einfache Resistenzmarker oder nicht-humane Selektionsmarker exprimiert. Eine besondere
Herausforderung stellt dagegen die Produktion von möglichst naturidentischen humanen Proteinen in nicht-Säugerzellen dar. Insbesondere dann, wenn es sich um glycosylierte Proteine handelt, wie z. B. EPO.

Beim Ciliaten *Tetrahymena* handelt sich um einen nicht pathogenen, einzelligen, eukaryotischen Mikroorganismus, der relativ nah verwandt zu höheren Eukaryoten ist und die dafür typischen Zelldifferenzierungen aufweist. Obwohl *Tetrahymena* ein echter, komplex differenzierter Eukaryot ist, ähnelt er in seinen Kultivierungs- und Wachstumseigenschaften aber eher den einfachen Hefen oder Bakterien und lässt sich gut auf relativ billigen Magermilchmedien im grossen Maßstab fermentieren. Unter optimalen Bedingungen beträgt die Generationszeit etwa 1,5-3 h und es können sehr hohe Zelldichten (2,2 x 10⁷ Zellen/ml, entsprechend 48 g/l Biotrockenmasse) erreicht werden (Kiy und Tiedke 1992, Appl. Microbiol. Biotechnol. 37: 576-579; Kiy und Tiedke 1992, Appl. Microbiol. Biotechnol. 38: 141-146). Dadurch ist *Tetrahymena* für eine fermentative Produktion von rekombinanten Proteinen im grossen Massstab sehr interessant und wesentlich vorteilhafter als beispielsweise Säugerzellen.

Um Aufschluss über die Glycolysierungsmuster der von *Tetrahymena* produzierten Proteine zu erhalten, wurden Proteinproben (aus dem Kulturmedium von *Tetrahymena-*Kulturen) mittels Lektinblots vor oder nach Verdau mit Peptid:N-Glycosidase F (PNGase F) untersucht (Methoden nach Ashfort & Platt, 1999, siehe oben). Darüber hinaus wurden die N-Glykane isoliert und mit Hilfe der FACE-Technologie (Fa. Glyko Inc.) sequenziert.

Die Untersuchungen mittels Lektinblot zeigen, dass einige Proteine der mikrosomalen bzw. der Medien-Fraktionen N-glykosyliert sind. In beiden Fraktionen konnten N-Glykane mit terminalen Mannosedisacchariden detektiert werden (PNGase F sensitive Bindung des Lektins GNA). Dabei handelt es sich um N-Glykane des Mannose-reichen Typs. Weitere PNGase F sensitive Lektinbindungen wurden nicht nachgewiesen, so dass das Vorkommen von terminalen Fucose- (Lektin AAA), Galaktose- (RCA, DSA) und N-Acetylglukosamin-Resten (DSA, WGA) in den N-Glykanen unwahrscheinlich ist. Die gefundene starke Glykosylierung der Medienproteine ist nicht auf die Kontamination durch das verwendete Medium zurückzuführen, da entsprechende Kontrollen keine Lektinbindung zeigten. Es handelt sich daher um von *Tetrahymena* sekretierte Proteine.

Vier der N-glykosidischen Oligosaccharide wurden isoliert und sequenziert. Es handelt sich dabei um folgende Strukturen: Man5GlcNAc2, Man4GlcNAc2, Man3GlcNAc2 und Man2GlcNAc2. Dabei handelt es sich um Teilstrukturen der Mannose-reichen N-glykosidischen Ketten, wie sie auch von Taniguchi et al. (J. Biol. Chem. 260, 13941-13946 (1985)) für *Tetrahymena pyriformis* nachgewiesen wurden. Hinweise auf komplexe oder hybride N-glykosidische Zuckerketten wurden nicht gefunden. Die Ergebnisse der Lektinblots wurden damit bestätigt.

Demnach zeigt *Tetrahymena* Glycosylierungen des einfachen Grundtyps (siehe Abbildung 1). Hinweise für O-glykosidische Ketten auf sekretierten Proteinen fehlen ganz. Gegenüber anderen Expressionssystemen besitzt *Tetrahymena* damit einen signifikanten Vorteil: Die Zuckerstrukturen sind den N-glykosidischen Strukturen der Säugerzellen wesentlich ähnlicher als z. B. bei Hefe (mannosereich mit viel mehr Mannoseresten und ungewöhnlicher Struktur) und Insekten und Pflanzen (Vorkommen von Xylose). Für einige Anwendungen könnte der einfache Glykosylierungstyp von *Tetrahymena* auch Vorteile gegenüber Säugerzellkulturen haben, die meist komplexe N-glykosidische Zuckerstrukturen produzieren, welche die biologische Aktivität des Proteins häufig stark beeinflussen (z.B. Clearing-rate etc.) und die Produktion erschweren (siehe z. B. Erythropoietin-Produktion, 80% falsche Glykosylierung). Damit eignet sich Tetrahymena ganz besonders zur Expression glycosylierter Proteine, wie beispielsweise Erythropoietin (EPO).

Die Transformation von *Tetrahymena* kann durch Mikroinjektion, Elektroporation oder Mikropartikelbeschuss erreicht werden. Dafür stehen eine Reihe von Vektoren, Promotoren etc. zur Verfügung. Die Selektion der Transformanten erfolgt mittels eines Resistenzmarkers. So wurde *Tetrahymena* z. B. erfolgreich mit einem rDNA-Vektor transformiert (Selektion durch eine Paromycin-Resistenz-Mutation der rRNA (Tondravi et al. 1986, PNAS 83:4396; Yu et al. 1989, PNAS 86: 8487-8491). In weiteren Transformationsexperimenten wurden erfolgreich Cycloheximid- oder Neomycin-Resistenzen in *Tetrahymena* exprimiert (Yao et al. 1991, PNAS 88:9493-9497; Kahn et al. 1993, PNAS 90: 9295-9299). Neben diesen Markergenen haben Gaertig et al. (1999, Nature Biotech. 17: 462-465) erfolgreich ein rekombinantes Fischparasiten-Antigen (aus einem Ciliaten) in *Tetrahymena* exprimiert. Weiters wurde von Gaertig et al. erfolgreich ein partielles Hühner Ovalbumin in *Tetrahymena* exprimiert (WO 00/46381 A1). Die Selektion erfolgte in beiden Fällen mit Hilfe von Paclitaxel (Taxol). Dieses von Gaertig et al. entwickelte System ist zum Patent angemeldet (WO 00/46381 A1).

Eine Integration des heterologen Gens durch homologe DNA-Rekombination ist möglich in *Tetrahymena.* Dadurch können mitotisch stabile Transformanten erzeugt werden. Durch die homologe DNA-Rekombination sind darüber hinaus auch gezielte Gen-Ausschaltungen (Gene Knockouts) möglich (Bruns & Cassidy-Hanley in: Methods in Cell Biology, Volume 62, Ed. Asai & Forney, Academic Press (1999) 501-512); Hai et al. in: Methods in Cell Biology, Volume 62, Ed. Asai & Forney, Academic Press (1999) 514-531; Gaertig et al. (1999) Nature Biotech. 17: 462-465 oder Cassidy-Hanley et al. 1997 Genetics 146:135-147). Ausserdem kann wahlweise der somatische Makronucleus oder der generative Mikronucleus transformiert werden. Bei der Makronucleustransformation erhält man sterile Transformanten, was aus Sicherheits- oder Akzeptanzfragen vorteilhaft sein kann.

In Anbetracht des Standes der Technik war es nun Aufgabe der vorliegenden Erfindung ein neues, einfach und wirtschaftlich durchzuführendes Herstellungsverfahren für humane Proteine zur Verfügung zu stellen, das die Möglichkeit zu posttranslationellen Proteinmodifikationen birgt, die möglichst dem Grundtyp der verschiedenen humanen Proteinmodifikationen entsprechen, das ferner bevorzugterweise die Sekretion der rekombinanten Proteine erlaubt, bevorzugt unter Verzicht auf den Einsatz nicht-menschlicher Sekretionssignale (Sekretionssequenzen).

Gelöst wird diese, sowie weitere nicht explizit genannte Aufgaben, die jedoch aus den hierin einleitend diskutierten Zusammenhängen ohne weiteres ableitbar oder erschliessbar sind, durch die in den Patentansprüchen definierten Ausführungsformen der vorliegenden Erfindung.

Ein Verfahren zur Herstellung von rekombinanten humanen Proteinen zur Verfügung zu stellen, gelingt auf erstaunlich einfache Art und Weise, indem man *Tetrahymena-*Zellen mit rekombinanter DNA, die mindestens ein funktionelles für ein humanes Protein codierendes Gen enthält, transformiert, die rekombinanten *Tetrahymena*-Zellen kultiviert, wobei das für ein humanes Protein codierende Gen exprimiert wird und anschliessend die Proteine isoliert werden. Insbesondere weisen die dergestalt produzierten rekombinanten humanen Proteine einfache Glycosylierungsmuster auf, deren Strukturen denen der Säugerzellen wesentlich ähnlicher sind als beispielsweise denen der Hefe. Kultiviefungs- und Wachstumseigenschaften dagegen entsprechen eher denen der Hefe, d. h. schnelles Wachstum auf billigen Medien, das ohne übermässigen technischen Aufwand zu erreichen ist.

Eine erfindungsgemäß bevorzugte Spezies der Gattung *Tetrahymena* ist *Tetrahymena thermophila.*

Unter einem humanen Protein versteht man erfindungsgemäß ein Protein für das eine entsprechende codierende DNA Sequenz aus dem Menschen isolierbar ist. Die schlussendlich für die rekombinante Expression verwendete DNA-Sequenz muss dabei nicht aus menschlichen Zellen isoliert worden sein, sie kann vielmehr auch in modifizierter Form vorliegen oder künstlich hergestellt worden sein. Das produzierte Protein kann auch gegenüber dem aus menschlichen Zellen isolierbaren Protein Mutationen, wie Deletionen, den Austausch bestimmter Aminosäuren und dergleichen mehr aufweisen. Insbesondere sind erfindungsgemäss mit humanen Proteinen, humane Proteine für einen therapeutischen Einsatz gemeint, wie Cytokine (Interferone, Interleukine), Enzyme, Hormone (Insulin, EPO, Wachstumshormone), Blutfaktoren (Faktor VIII, Faktor IX) und weitere.

Unter einfachen Glycosylierungsmustern versteht man für die Zwecke der vorliegenden Erfindung haupsächlich Glycosylierungsmuster der Grundstruktur GlcNAc2-Man2-5.

Unter einem funktionellen Gen versteht man für die Zwecke der vorliegenden Erfindung ein Gen, das im Zielorganismus exprimiert werden kann. Insbesondere umfasst ein funktionelles Gen daher neben einer codierenden Sequenz, einen im Zielorganismus funktionellen Promotor, der zur Transkription der codierenden Sequenz führt. Ein dergestalt funktioneller Promotor kann unter anderem eine oder mehrere TATA-Boxen, CCAAT-Boxen, GC-Boxen oder Enhancer-Sequenzen aufweisen. Weiters kann das funktionelle Gen einen im Zielorganismus funktionellen Terminator umfassen der zum Abbruch der Trankription führt und Signalsequenzen enthält, die zur Polyadenylierung der mRNA führen. Die codierende Sequenz des funktionellen Gens weist ferner alle für die Translation im Zielorgansimus notwendigen Eigenschaften auf (beispielsweise Start-Codon (z. B. ATG), Stop-Codon (TGA), A-reiche Region vor dem Start (Translation initiation site), Kozak-Sequenzen, Poly-A Stelle. Das Gen kann auch die für *Tetrahymena* spezifische Codonusage aufweisen (Wuitschick & Karrer, J. Eukaryot. Microbiol. (1999)).

In einer bevorzugten Ausführungsform der vorliegenden Erfindung handelt es sich bei dem exprimierten Gen um ein Gen für humanes Serum Albumin (HSA) ganz besonders bevorzugt um das Gen mit der GenBank Accession No.: 8392890 oder eine Variante davon. Zum Thema GenBank siehe Benson, D.A. et al., Nuc. Acid Res., 28 (T), 15-18 (2000). Das hergestellte Protein ist demgemäß bevorzugterweise humanes Serum Albumin.

Unter einer Variante von rekombinantem HSA versteht man erfindungsgemäss ein Gen mit mindestens 70%, bevorzugt 80%, besonders bevorzugt 90% und ganz besonders bevorzugt 95% Homologie zur Sequenz des Gens mit der GenBank Accession No.: 8392890. Insbesondere hybridisiert eine Sonde von bevorzugt 100 bis 300 bp ausgewählt aus der Sequenz des Gens mit der GenBank Accession No.: 8392890 unter Standard-Hybridisierungs-Bedingungen an eine Variante dieses Gens.

In der vorliegenden Arbeit wird zum erstenmal die erfolgreiche Expression von rekombinantem HSA in *Tetrahymena* beschrieben und damit zum ersten Mal überhaupt, dass ein humanes Protein erfolgreich in *Tetrahymena* exprimiert werden konnte.

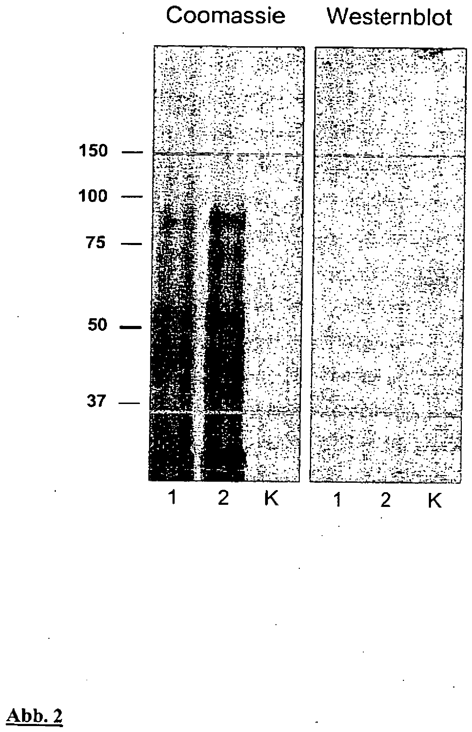

Dazu wurde die vollständige kodierende Sequenz von HSA einschliesslich der Leader-Sequenz durch PCR amplifiziert und in einen *Tetrahymena-*Expressionsvektor ligiert (siehe Bsp. 2). Nach der Transformation von *Tetrahymena* mit diesem Konstrukt und Selektion der Transformanten auf Taxol-Resistenz (siehe Bsp. 3), wurden Kulturen in Magermilchmedium unter Zusatz von Protease-Inhibitor angezogen (siehe Bsp. 5). Von *Tetrahymena* ins Medium sekretierte Proteine wurden mittels SDS-PAGE aufgetrennt und HSA mit einem HSA-spezifischen Antikörper im Westernblot nachgewiesen (siehe Abb. 2). Erstaunlicherweise gelang es nicht nur, das rekombinante HSA zu produzieren, sondern auch ins Medium zu sekretieren.

Entgegen aller Erwartungen erwies sich die humane Leader-Sequenz als voll funktionsfähig in *Tetrahymena.*

*Tetrahymena*-Zellen die das exprimierte Protein sekretieren, bilden eine bevorzugte Ausführungsform der vorliegenden Erfindung. Die Isolierung der Proteine kann bei dieser Ausführungsform aus dem Kulturüberstand erfolgen.

Eine ganz besonders bevorzugte Ausführungsform der vorliegenden Erfindung betrifft daher Verfahren zur Herstellung von rekombinanten humanen Proteinen wobei das für das humane Protein codierende Gen eine humane Leader-Sequenz umfasst, die die Sekretion des exprimierten Proteins von *Tetrahymena* bewirkt. Für eine Beschreibung von Leader-Sequenzen (synonym mit Signalsequenzen), bzw. Leader-Peptiden, (oder Signal-Peptiden) siehe beispielsweise Stryer, L., Biochemie, 4. Aufl., Spektrum Akademischer Verlag, 1996, Seiten 802-3, oder Alberts, B. et al., Molecular Biology of the Cell. 3rd ed. Garland Publishing, New York und London, 1994, Chapter 12 oder Lodish, H. et al., Molecular Cell Biology, 4th ed., W H Freeman & Co. New York, 1999, Chapter I7. Ganz besonders bevorzugt umfasst die Leader-Sequenz die Nukleotid-Sequenz atgaagtggg taacctttat ttcccttctt tttctcttta gctcggctta ttcc (SEQ ID No. 5), oder kodiert für ein Leader Peptid umfassend die Aminosäure-Sequenz Met Lys Trp Val Thr Phe Ile Ser Leu Leu Phe Leu Phe Ser Ser Ala (SEQ ID No. 6). In einer anderen bevorzugten Ausführungsform umfasst die Leader-Sequenz eine Sequenz entsprechend der Propeptid-Sequenz aggggtgtgt ttcgtcga (SEQ ID No. 7), oder kodiert für ein Propeptid umfassend die Aminosäure-Sequenz Arg Gly Val Phe Arg Arg (SEQ ID No. 8).

In einer anderen Ausführungsform der vorliegenden Erfindung wird das humane Protein auf der Oberfläche der rekombinanten *Tetrahymena*-Zellen exprimiert.

Eine weitere Offenbarung betrifft rekombinante *Tetrahymena-*Zellen, die DNA enthalten, die für ein humanes Protein codiert. Bevorzugterweise exprimieren die rekombinanten *Tetrahymena*-Zellen das rekombinante humane Gen. Besonders bevorzugt umfasst das exprimierte Gen eine humane Leader-Sequenz. Bevorzugterweise umfasst besagte Leader-Sequenz SEQ ID No. 5, oder kodiert für ein Leader Peptid umfassend SEQ ID No. 6. In einer anderen bevorzugten Ausführungsform umfasst die Leader-Sequenz eine Sequenz entsprechend der Propeptid-Sequenz SEQ ID No. 7, oder kodiert für ein Propeptid umfassend SEQ ID No. 8.

Ganz besonders bevorzugt sekretieren die rekombinanten *Tetrahymena*-Zellen das rekombinante humane Protein. In einer bevorzugten Ausführungsform handelt es sich bei dem dergestalt exprimierten und sekretierten Protein um humanes Serum Albumin, insbesondere um das Expressionsprodukt des Gens mit der GenBank Accession No.: 8392890 oder einer Variante davon.

Eine weitere Ausführungsform der vorliegenden Erfindung betrifft ein rekombinantes humanes Glycoprotein, dadurch gekennzeichnet, dass es ein für *Tetrahymena* typischen Glycosylierungsmuster mit der Grundstuktur GlcNAc2.Man2-5 aufweist und durch eine *Tetrahymena*-Zelle exprimiert wurde.

Ein Expressionsvektor zur Expression humaner Proteine in *Tetrahymena*, umfassend eine humane Leader-Sequenz bildet eine weitere Offenbarung.

Unter einem Expressionsvektor versteht man erfindungsgemäß ein Nukleinsäuremolekül, wie DNA oder RNA, zirkulär oder linear, beispielsweise ein Plasmid, ein Cosmid oder ein künstliches Chromosom, das es ermöglicht, ein rekombinantes Gen in eine Wirtszelle einzuschleusen und das Gen in der Zelle zu exprimieren. Der Vektor kann in der Zelle episomal vorliegen, d.h. selbstreplizierend sein oder ins Genom der Wirtszelle integriert werden. Die Integration kann zufällig oder auch durch homolge Rekombination erfolgen. Ausser dem rekombinanten zu exprimierenden Gen kann ein solcher Expressionsvekor erfindungsgemäss andere für die Aufgabe hilfreiche Sequenzen umfassen, wie multiple cloning sites, autonomously replicating sequences, Markergene für den Wirt als auch alle notwendigen Sequenzen um Replikation in *E. coli* zu Klonierungszwecken zu ermöglichen, wie einen ori, *E. coli* spezifische Marker etc.

In der vorliegenden Arbeit wird zum erstenmal die erfolgreiche Expression eines humanen Proteins in *Tetrahymena* beschrieben.

### Beschreibung der Abbildungen:

Abbildung 1: N-gebundene Proteinglycosylierung
   Mannosereicher Typ: mit 5-9 Mannose- und 2 GlcNAc-Resten
   Einfache Grundstruktur: mit 2-5 Mannose- und 2 GlcNAc-Resten
   Komplexer Typ: mit Galactose-, Fucose- und Sialinsäureresten (N-Acetylneuraminsäure)
   Hefe N-Glycane bestehen ausschliesslich aus dem mannosereichen Typ mit 8-50 Mannoseresten.
   Der pflanzlich komplexe Typ weist keine Sialinsäure auf, kann aber Xylosereste (allergenes Potential!) besitzen.
Abbildung 2: Expression von HSA in *Tetrahymena*
   Spur 1 und 2: Medienfraktion von *Tetrahymena,* transformiert mit pBHSA nach 24 und nach 48 h, Spur K: natürliches HSA als Kontrolle. SDS-PAGE, gefärbt mit Coomassie-Blue und Western-Blot mit HSA-Antikörper und sekundärem Antikörper (gekoppelt an Alkalische Phosphatase

### Beispiele:

Die nachfolgenden Beispiele dienen zur Erläuterung der Erfindung, ohne die Erfindung auf diese Beispiele einzugrenzen

### Beispiel 1: Organismen und Kultivierungsbedingungen

*Tetrahymena thermophila* (Stämme B1868 VII, B2086 II, B*VI, CU428, CU427, CU522, zur Verfügung gestellt von J. Gaertig, University of Georgia, Athens, GA, USA) wurden in modifiziertem SPP-Medium (2% Proteosepeptone, 0,1% Hefeextrakt, 0,2% Glucose, 0,003% Fe-EDTA (Gaertig et al. (1994) PNAS 91:4549-4553)) bzw. Magermilchmedium (2% Magermilchpulver, 0,5% Hefeextrakt, 1% Glucose, 0,003% Fe-EDTA) oder MYG-Medium (2% Magermilchpulver, 0,1% Hefeextrakt, 0,2% Glucose, 0,003% Fe-EDTA) mit Zusatz von Antibiotika-Lösung (100 U/ml Penicillin, 100 µg/ml Streptomycin und 0,25 µg/ml Amphotericin B (SPPA-Medium)) bei 30 °C in 50 ml Volumen in 250 ml Erlenmeyerkolben unter Schütteln (150 U/min) kultiviert.

Plasmide und Phagen wurden in *E. coli* XL1-Blue, MRF', TOP10F' oder JM109 (Stratagene, Invitrogen, GibcoBRL Life Technologies) vermehrt und selektiert. Die Kultivierung der Bakterien erfolgte unter Standardbedingungen in LB- oder NZY-Medium, mit Antibiotika in Standardkonzentrationen (Sambrook et al. (1989) MolecularCloning: A Laboratory Manual, Cold Spring Harbor Laboratory, Cold Spring, New York).

### Beispiel 2: Herstellung des Expressionskonstruktes pBHSA

Der Vektor pBICH3 (Gaertig et al. 1999 Nature Biotech. 17: 462-465, WO 00/46381 A1) enthält die kodierende Sequenz des *Ichthyophthinius* I-Antigen (G1) Präprotein flankiert von den nicht kodierenden, regulativen Sequenzen des *Tetrahymena thermophila* BTU1-Gens. Ein modifiziertes Plasmid (pBICH3-Nsi) mit einer Nsi I Schnittstelle am Start (zur Verfügung gestellt von J. Gaertig, University of Georgia, Athens, GA, USA) wurde benutzt, um das humane Serum Albumin (HSA, GenBank Accession No.: 8392890) Expressionskonstrukt pBHSA herzustellen. Dazu wurden mittels PCR Nsi I und Bam HI Schnittstellen am Start und Stop der kodierenden HSA-Sequenz eingefügt. Für die PCR wurde ein isoliertes Plasmid (ATCC 323324), welches die vollständigen cDNA-Sequenzen von HSA enthält als Template eingesetzt. Die Primer
HSA-Nsi-1 5' GGCACAATGCATTGGGTAACCTTTATTAGC-3' (SEQ ID No. 1) und
HSA-Bam-R 5'- AAATGGGATCCTCATAAGCCTAAGGCAGCTTGAC-3' (SEQ ID No. 2)
erzeugten ein PCR-Produkt, welches die vollständige kodierende Sequenz des HSA, flankiert von Nsi I und Bam HI Schnittstellen, enthielt. Das PCR-Produkt und das Plasmid pBICH3-Nsi wurden mit den Restriktionsenzymen Nsi I und Bam HI geschnitten, über ein Agarosegel gereinigt und ligiert. Das so entstandene Expressionskonstrukt pBHSA enthielt die vollständige HSA kodierende Sequenz inseriert im korrekten Leseraster in die regulatorischen Sequenzen des BTU1 Gens. Für die Transformation von *Tetrahymena* wurde dieses Konstrukt durch einen Verdau mit den Restriktionsenzymen Sac II und Xho I linearisiert

Bei einer erfolgreichen Transformation wurde durch homologe Rekombination das BTU1-Gen durch dieses Konstrukt ersetzt, wodurch eine Resistenz der Zellen gegenüber Paclitaxel vermittelt wurde.

### Beispiel 3: Macronucleus-Transformation von Tetrahymena mit pBHSA

Für eine Transformation wurden 5 x 10⁶ *Tetrahymena thermophila* Zellen (CU522) eingesetzt. Die Kultivierung der Zellen erfolgte in 50 ml SPPA-Medium bei 30 °C in einem 250 ml Erlenmeyerkolben auf einem Schüttler bei 150 RPM bis zu einer Zelldichte von ca. 3-5 x 10⁵ Zellen/ml. Die Zellen wurden durch Zentrifugation (1200 g) für 5 min pelletiert und das Zellpellet wurde in 50 ml 10 mM Tris-HCl (pH 7,5) resuspendiert und wie vorher zentrifugiert. Dieser Waschschritt wurde wiederholt und die Zellen in 10 mM Tris-HCl (pH 7,5, plus Antibiotika) bei einer Zelldichte von 3 x 10⁵ Zellen/ml resuspendiert, in einen 250 ml Erlenmeyerkolben überführt und für 16-20 h ohne SchütteIn bei 30 °C inkubiert (Hungerphase). Nach der Hungerphase wurde erneut die Zellzahl bestimmt, wie oben zentrifugiert und die Zellen mit 10 mM Tris-HCl (pH 7,5) auf eine Konzentration von 5 x 10⁶ Zellen/ml eingestellt. Ein ml der Zell-Suspension wurde für die Transformation benutzt. Die Transformation erfolgte mittels Mikropartikelbeschuss (s. u.). Zur Regeneration wurden die Zellen in SSPA-Medium aufgenommen und bei 30 °C ohne Schütteln im Erlenmeyerkolben inkubiert. Nach 3 h wurde Paclitaxel^{®} in einer Endkonzentration von 20 µM zugegeben und die Zellen in Aliquots von 100 µl auf 96er Mikrotiterplatten überführt. Die Zellen wurden in einer feuchten, abgedunkelten Box bei 30 °C inkubiert. Nach 2-3 Tagen konnten Paclitaxel resistente Klone identifiziert werden. Positive Klone wurden in frisches Medium mit 25 µM Paclitaxel überimpft. Durch Kultivierung der Zellen in steigender Paclitaxel-Konzentration (bis 80 µM) wurde ein komplettes "phenotypic assortment" (Gaertig & Kapler (1999)) erreicht.

Zur Analyse der Klone wurden ca. 4 ml Kulturen in SPPA mit Paclitaxel angezogen, DNA isoliert (Jacek Gaertig et al. (1994) PNAS 91:4549-4553) und durch PCR die in den BTU1-Locus integrierte DNA amplifiziert. Als Primer dienten die BTU1 spezifischen Primer
BTU1-5'F (AAAAATAAAAAAGTTTGAAAAAAAACCTTC SEQ ID No. 3), ca. 50 bp vor dem Startcodon und

BTU1-3'R (GTTTAGCTGACCGATTCAGTTC (SEQ ID No. 4), 3 bp hinter dem Stopcodon. Die PCR-Produkte wurden ungeschnitten und mit Hind III, Sac I oder Pst I geschnitten auf einem 1%igen Agarosegel analysiert. Vollständiges "phenotypic assortment" wurde über RT-PCR mit den BTU1 spezifischen Primern (Gaertig & Kapler (1999)) überprüft.

### Beispiel 4: Biolistische Transformation (Mikropartikelbeschuss)

Die Transformation von *Tetrahymena thermophila* erfolgte mittels biolistischer Transformation, wie sie bei Bruns & Cassidy-Hanley (Methods in Cell Biology, Volume 62 (1999) 501-512); Gaertig et al. (1999) Nature Biotech. 17: 462-465) oder Cassidy-Hanley et al. ((1997) Genetics 146:135-147) beschrieben ist. Die Handhabung des Biolistic^{®} PDS-1000/He Particle Delivery System (BIO-RAD) ist detailliert im zugehörigen Handbuch beschrieben.

Für die Transformation werden 6 mg Goldpartikel (0,6 mu m; BIO-RAD) mit 10 µg linearisierter Plasmid DNA beladen (Sanford et al. (1991) Biotechniques 3:3-16; Bruns & Cassidy-Hanley (1999) Methods in Cell Biology, Volume 62: 501-512).

Vorbereitung der Goldpartikel: 60 mg der 0,6 µm Goldpartikel (Biorad) wurden in 1 ml Ethanol resuspendiert. Dazu wurden die Partikel 3 mal für je 1-2 min auf einem Vortex kräftig gemixt. Anschliessend wurden die Partikel für 1 min zentrifugiert (10000 g) und der Überstand vorsichtig mit einer Pipette abgenommen. Die Goldpartikel wurden in 1 ml sterilem Wasser resuspendiert und wie oben zentrifugiert. Dieser Waschschritt wurde einmal wiederholt, die Partikol in 1 ml 50%igem Glycerol resuspendiert und in Aliquots zu 100 µl bei -20 °C gelagert.

Vorbereiten der Transformation: Die Macrocarrierhalter, Macrocarrier und Stopscreens wurden für mehrere Stunden in 100% Ethanol, die Rupture Disks in Isopropanol gelagert. Ein Macrocarrier wurde anschliessend in den Macrocarrier-Halter eingesetzt und an der Luft getrocknet.

Beladung der Goldpartikel mit DNA: Alle Arbeiten erfolgten bei 4 °C. Goldpartikel, vorbereiteter Vektor, 2,5 M CaCl₂, 1 M Spermidine, 70% und 100% Ethanol wurden auf Eis gekühlt. 10 mu 1 der linearisierten Vektor-DNA (1 µg/ml) wurden zu 100 µl vorbereiteten Goldpartikeln gegeben und für 10 sec vorsichtig gevortext. Anschliessend wurden erst 100 µl 2,5 M CaCl₂ zugegeben, 10 sec gevortext und dann 40 µl 1 M Spermidine zugegeben und 10 min vorsichtig gevortext. Nach Zugabe von 200 mu 1 70%igem Ethanol wurden die Partikel für 1 min gevortext und dann 1 min bei 10000 g zentrifugiert. Das Pellet wurde in 20 µl 100%igem Ethanol resuspendiert, zentrifugiert und dann in 35 µl 100%igem Ethanol resuspendiert.

Die so vorbereiteten Partikel wurden vorsichtig mit einer Pipette auf das Zentrum eines Macrocarriers gegeben. Der Macrocarrierer wurde anschliessend bis zur Transformation in einer Box mit hygroskopischen Silicagel gelagert.

Transformation: Ein ml der vorbereiteten Zellen (siehe oben) wurde in die Mitte eines mit 10 mM Tris-HCl (pH 7,5) angefeuchteten Rundfilters in einer Petrischale gegeben und in die unterste Einschubleiste der Transformationskammer des Biolistic^{®} PDS-1000/He Particle Delivery Systems eingesetzt. Die Transformation erfolgte mit den vorbereiteten Goldpartikeln bei einem Druck von 900 psi (zwei 450 psi Rupture Disks) und einem Vakuum von 27 inches Hg in der Transformationskammer. Anschliessend wurden die Zellen sofort in einen Erlenmeyerkolben mit 50 ml SPPA-Medium überführt und bei 30 °C ohne Schütteln inkubiert.

### Beispiel 5: Expression von HSA in Tetrahymena

Von positiven Transformanten und als Kontrolle von nicht transformierten *Tetrahymena*-Wildtypzellen wurden Kulturen in Erlenmeyerkolben in 20 ml Magermilchmedium unter Zusatz von Protease-Inhibitor (Complete, EDTA-free Protease-Inhibitoren-Cocktail Tabletten, Roche Diagnostics GmbH) angezogen. Nach 24 h und 48 h (bei einer Zelldichte von ca. 1 x 10⁶ Zellen/ml) wurden die Zellen abzentrifugiert und der Überstand mit 1/10 Volumen eiskaltem TCA versetzt und 30 min auf Eis inkubiert und anschliessend für 30 min bei 4 °C bei max. rpm zentrifugiert. Das Pellet wurde mit 300 µl eiskaltem Aceton gewaschen und 5 min bei 4 °C bei max. rpm zentrifugiert. Der Überstand wurde abgenommen, das Pellet getrocknet (Vakuum), in 150 µl Probenpuffer resuspendiert und 10 min bei 95 °C inkubiert. Die so gewonnenen Proteine aus der Medien-Fraktion, von *Tetrahymena* ins Medium sekretierter Proteine, wurden mittels SDS-PAGE nach Standard-Methoden aufgetrennt und HSA mit einem HSA-spezifischen Antikörper (Sigma) im Westernblot nachgewiesen (siehe Abb. 2). Als positiv-Kontrolle wurde natürliches HSA (Sigma) verwendet.
## Patentansprüche

1. Verfahren zur Herstellung von rekombinanten humanen Glycoproteinen in *Tetrahymena* umfassend die Stufen:
a) Transformation von *Tetrahymena*-Zellen mit rekombinanter DNA, die mindestens ein funktionelles, für ein humanes Glycoprotein codierendes Gen umfasst,
b) Kultivierung der rekombinanten Tetrahymena-Zellen und Expression des Gens, und
c) Isolierung der Glycoproteine.

2. Verfahren zur Herstellung von rekombinanten humanen Glycoproteinen gemäß Anspruch 1, wobei die *Tetrahymena*-Zellen das Glycoprotein sekretierten.

3. Verfahren zur Herstellung von rekombinanten humanen Glycoproteinen gemäß Anspruch 1, wobei die *Tetrahymena*-Zellen das Glycoprotein auf ihrer Oberfläche exprimieren.

4. Verfahren zur Herstellung von rekombinanten humanen Glycoproteinen gemäß Anspruch 2, wobei die Isolierung der Glycoproteine aus dem Kulturüberstand erfolgt.

5. Verfahren zur Herstellung von rekombinanten humanen Glycoproteinen nach einem der vorstehenden Ansprüche, wobei das für ein humanes Glycoprotein codierende Gen eine humane Leader-Sequenz umfasst.

6. Verfahren zur Herstellung von rekombinanten humanen Glycoproteinen gemäß Anspruch 5, wobei die humane Leader-Sequenz zur Sekretion des exprimierten Glycoproteins von *Tetrahymena* führt.

7. Rekombinantes humanes Glycoprotein, **dadurch gekennzeichnet, dass** es ein Glycosylierungsmuster mit der Grundstruktur GlcNAc2-Man2-5 aufweist und durch eine *Tetrahymena*-Zelle exprimiert wurde.

8. Verwendung eines humanen rekombinanten Glycoproteins zur Herstellung eines Arzneimittels, **dadurch gekennzeichnet, dass** das Glycoprotein in *Tetrahymena*-Zellen exprimiert und daraus isoliert worden ist.

## Claims

1. Process for manufacturing recombinant human Glycoproteins in *Tetrahymena* comprising the steps of:
a) Transformation of *Tetrahymena* cells with recombinant DNA which comprises at least one functional gene encoding a human glycoprotein,
b) Cultivating the recombinant *Tetrahymena* cells and expression of the genes, and
c) Isolation of the glycoproteins.

2. Process for manufacturing recombinant human Glycoproteins according to claim 1, wherein the *Tetrahymena* cells secrete the glycoprotein.

3. Process for manufacturing recombinant human Glycoproteins according to claim 1, wherein the *Tetrahymena* cells express the glycoprotein on their surface.

4. Process for manufacturing recombinant human Glycoproteins according to claim 2, wherein the isolation of the glycoproteins occurs from the culture supernatant.

5. Process for manufacturing recombinant human glycoproteins according to one of the preceding claims, wherein the gene encoding for a human glycoprotein comprises a human leader sequence.

6. Process for manufacturing recombinant human Glycoproteins according to claim 5, wherein the human leader sequence leads to secretion of the expressed glycoprotein from *Tetrahymena.*

7. Recombinant human glycoprotein, **characterized in that** it possesses a glycosylation pattern with the base structure GlcNAc2-Man2-5 and was expressed by a *Tetrahymena* cell.

8. Use of a human recombinant glycoprotein for manufacturing a medicament, **characterized in that** the glycoprotein was expressed in *Tetrahymena* cells and isolated therefrom.

## Revendications

1. Procédé de préparation de glycoprotéines humaines recombinantes dans *Tetrahymena,* comprenant les étapes :
a) de la transformation de cellules de *Tetrahymena* avec de l'ADN recombinant, qui comprend au moins un gène fonctionnel, codant pour une glycoprotéine humaine,
b) de la culture des cellules recombinantes de *Tetrahymena* et de l'expression du gène, et
c) de l'isolation des glycoprotéines.

2. Procédé de préparation de glycoprotéines humaines recombinantes selon la revendication 1, les cellules de *Tetrahymena* sécrétant la glycoprotéine.

3. Procédé de préparation de glycoprotéines humaines recombinantes selon la revendication 1, les cellules de *Tetrahymena* exprimant la glycoprotéine sur leur surface.

4. Procédé de préparation de glycoprotéines humaines recombinantes selon la revendication 2, l'isolation des glycoprotéines s'effectuant à partir du surnageant de culture.

5. Procédé de production de glycoprotéines humaines selon l'une quelconque des revendications précédentes, le gène codant pour une glycoprotéine humaine comprenant une séquence leader humaine.

6. Procédé de préparation de glycoprotéines humaines recombinantes selon la revendication 5, la séquence humaine leader conduisant à la sécrétion de la glycoprotéine exprimée de *Tetrahymena.*

7. Glycoprotéine humaine recombinante, **caractérisée en ce qu'**elle présente un motif de glycosylation avec la structure de base GlcNAc2-Man2-5 et a été exprimée par une cellule *Tetrahymena.*

8. Utilisation d'une glycoprotéine humaine recombinante pour la production d'un médicament, **caractérisée en ce que** la glycoprotéine a été exprimée dans des cellules de Tetrahymena et a été isolée à partir de celles-ci.
